# EUROPEAN PATENT APPLICATION

(11) **EP 2 343 310 A1**
(43) Date of publication of application: **13.07.2011**
(21) Application number: 10150333.2
(22) Date of filing: 08.01.2010
(51) Int. Cl.: C07K 5/08, C11D 3/16, C11D 3/20, C11D 3/26, C11D 3/386, A61K 38/46, A61K 38/06, A61K 31/11, A61K 31/69

(54) **Serine hydrolase formulation**

(71) Applicant: Novozymes A/S, 2880 Bagsvaerd (DK)
(72) Inventor: The designation of the inventor has not yet been filed

(57) **Abstract**

The storage stability of a serine hydrolase acting on ester bonds in a formulation without a protease can be improved by incorporating a serine protease inhibitor.

## Description

### FIELD OF THE INVENTION

The present invention relates to a composition which comprises a serine hydrolase acting on ester bonds and which does not comprise a protease.

### BACKGROUND OF THE INVENTION

Serine hydrolases acting on ester bonds such as lipases for industrial use may be supplied in a liquid or granule formulation for use, e.g., in liquid or granular detergents. The formulation is typically free of protease and/or free of surfactant, and it is generally desirable to improve the storage stability of the serine hydrolase in the formulation.

WO 2009/118375 discloses that in a liquid detergent containing a protease and a lipase, the storage stability of the lipase can be improved by the addition of a peptide aldehyde due to inhibition of the protease.

### SUMMARY OF THE INVENTION

The inventors have found that the storage stability of a serine hydrolase acting on ester bonds in a formulation without a protease can be improved by incorporating a serine protease inhibitor. Without wishing to be bound by theory, the inventors believe that the structure of the serine hydrolase is stabilized by incorporating a serine protease inhibitor in the active site.

Accordingly, the invention provides a composition which comprises a serine hydrolase acting on ester bonds and a serine protease inhibitor, and which does not comprise a protease.

### DETAILED DESCRIPTION OF THE INVENTION

### Serine hydrolase acting on ester bonds

The serine hydrolase is an enzyme in the serine hydrolase superfamily. It acts on ester bonds and belongs to enzyme class EC 3.1. It may be a lipase (EC 3.1.1.3), a phospholipase A2 (EC 3.1.1.4), a carboxylesterase (EC 3.1.1.1), an acetylcholinesterase (EC 3.1.1.7), or a thioester hydrolase (EC 3.1.2).

The lipase may be *Thermomyces lanuginosus* lipase (TLL, shown as SEQ ID NO: 2 in WO 2009/109500), Candida *antarctica* lipase A (CALA, shown as SEQ ID NO: 1 in WO 2008/040739), *Candida antarctica* lipase B (CALB, having the amino acid sequence described in J. Uppenberg et al., Structure, 1994, 2:293-308), *Rhizomucor miehei* lipase, *Aspergillus niger* lipase, *Candida rugosa* lipase (SWISSPROT:P20261), *Penicillium roqueforti* lipase, *Rhizopus niveus* lipase (SWISPROT:P61872), *Rhizopus oryzae* lipase, *Alcaligenes sp.* lipase, *Achromo-bacter sp*. lipase, *Burkholderia cepacia* lipase, *Pseudomonas stutzeri* lipase, or it may be a variant which has an amino sequence with at least 80% identity to one of these, particularly at least 85%, at least 90%, at least 95% or at least 98% identity.

Examples of TLL homologues are described in WO 1992/005249 (NZ 3520, Lipolase Ultra), W00060063, W09707202, W00032758, W002055679, W004099400, W007087508 and WO 2009/109500. Commercial lipases include the following products of Novozymes A/S: Novozym^{™} 435, Novozym 735, Lipozyme^{™} RM, Novozym 388, Lipolase Ultra^{™} , Lipex^{™}, Lipoprime^{™}, Resinase^{™} HT, Lipolase^{™}, Resinase^{™}, Lipopan^{™}, Lipoclean^{™} and Lipolex^{™}. Commercial lipases also include the following products of Amano Enzyme, Danisco, DSM, Meito Sangyo: Lipase A, LipaseG, Lipase AY, Lipase R, Newlase^{™} F, Grindamyl^{™}, Powerbake^{™}, BakeZyme L, BakeZyme PH, Panamore, Panamore Golden, Panamore Spring, Lipase MY, Lipase OF, Lipase PL, Lipase QL, Lipase AL, Lipase SL, Lipase TL, and Lipase UL.

The phospholipase A2 may be of animal origin, e.g. pancreatic such as porcine pancreatic or bovine pancreatic. The phospholipase A2 may also be derived from other eukatyotic organisms such as *Tuber, Verticillium, Neurospora, Aspergillus, Helicosporum* or *Fusarium,* particularly *T. borchii, T. albidum, V. dahliae, V. tenerum, N. crassa, A. oryzae, Helicosporium* sp.HN1 or *F. venenatum,* or it may be a variant having a sequence identity of at least 80%, 85%, 90% or 95% to one of those mentioned, e.g. as described in W004097012.

### Serine protease inhibitor

The serine protease inhibitor is selected from inhibitors to proteases from the serine hydrolase superfamily. In particular, the serine protease inhibitor may be a peptide aldehyde or a boronic acid derivative.

The inhibitor may have an inhibition constant to a serine protease Ki (M, mol/L) of 1 E-12 - 1 E-03; more preferred 1E-11 - 1 E-04; even more preferred: 1E-10 - 1 E-05; even more preferred 1 E-10 - 1 E-06; most preferred 1 E-09 - 1 E-07.

The serine protease inhibitor has the general formula A⁴-A³-A²-A¹, where
- A¹ is an aldehyde (CHO) or aldehyde analogue (COCX₃, COCHX₂, COCH₂X or COCH₃, where X is a halogen atom), a phosphorous derivative or a boronic acid (B(OH)₂) with a possibility to bind to the serine in the active site of the serine hydrolase.
- A² may be an aromatic, or an aliphatic part, which might be an optionally substituted phenyl, an aliphatic chain with at least 2 carbons (optionally branched with aliphatic or aromatic substituents), or an amino acid residue with aromatic or aliphatic side chain. If A² is an amino acid, then the C-terminal carbon is included in A¹ described above. Preferred amino acids are D- or L-Tyr (p-tyrosine), m-tyrosine, 3,4-dihydroxy-phenylalanine, Leu, Phe, Val, Met, Nva, Nle and preferred phenyl derivatives are phenyl, 2-, 3- or 4-formylphenyl. Preferred aliphatic derivatives are -CH2-CH(R")- with R"= H, CH₃, CH₂Ph, CH(CH₃)₂, CH₂C₆H₄-pOH and other amino acid side chain analogues). When A¹ is a boronic acid, A² may also be OH yielding boric acid/borax.
- A³ if present may a non-bulky linker, preferably hydrophobic, consisting of either 1-3 small amino acids (ex Gly, Ala, Leu, norleucine, norvaline) or a substituted/unsubstituted aliphatic chain with 1-12 atoms, preferably carbon, oxygen and nitrogen, most preferred 4-10 atoms.
- A⁴ if present may be a protection group (e.g. an N-capping group as described below), any amino acid, a small or a bulky group.

The serine protease inhibitor may be a peptide aldehyde or an aldehyde analogue having the formula B²-B¹-B⁰-R wherein:
a) R is hydrogen, CH₃, CX₃, CHX₂, or CH₂X, wherein X is a halogen atom; a phosphonate or a boronic acid (B(OH)2)..
b) B⁰ is a single amino acid residue;
c) B¹ is 1-2 amino acid residues; and
d) B² consists of one or more amino acid residues (preferably one or two), optionally comprising an N-terminal protection group or an N-terminal protection group attached to B¹ (preferred if B¹ is a dipeptide).

In the above formula, B⁰ may be an L or D-amino acid with an optionally substituted aliphatic or aromatic side chain, preferably D- or L-Tyr (p-tyrosine), m-tyrosine, 3,4-dihydroxyphenylalanine, Leu, Phe, Val, Gly, Met, Trp, Ile, Nva or Nle. If R is boronic acid or a phosphonate then B⁰ is an amino acid residue without double-bound oxygen on the C-terminal carbon.

B¹ may be 1-2 amino acid residues, preferably 2, with a small optionally substituted aliphatic side chain, preferably selected from Ala, Cys, Gly, Leu, Arg, Pro, Ser, Ile, Thr, Val, Nva, or Nle, most preferred selected from Ala, Gly, Leu, Nva or Nle. The most preferred combinations are, Gly-Ala, Ala-Ala, Gly-Gly, and Gly-Leu.

B² may be an N-capping group known from protein chemistry, preferably selected from benzyloxycarbonyl (Cbz), p-methoxybenzyl carbonyl (MOZ), benzyl (Bn), benzoyl (Bz), p-methoxybenzyl (PMB), p-methoxyphenyl (PMP), formyl, acetyl, methyloxy, methyloxycarbonyl/methyl carbamate, or methyl urea. or B2 may be any1-2 amino acid residues without specifying structure optionally comprising a protection group as described above.

More particularly, the peptide aldehyde may be Z-GGY-H, Z-GGF-H, Z-GGG-H, Z-GAG-H, Z-RAY-H, Ac-GAY-H, Z-GAY-H, Z-GAL-H, Z-GAF-H, Z-GAV-H, Z-RVY-H, Z-LVY-H, Ac-LGAY-H, Ac-FGAY-H, Ac-YGAY-H, Ac-FGVY-H, Ac-FGAM-H, Ac-WLVY-H, MeO-CO-VAL-H, MeO-CO-LLY-H, MeOCO-FGAL-H, MeO-FGAF-H, MeNCO-FGAL-H Z-VAL-CF₃, Ac-NHCH(CH₂CH(CH₃))-B(OH)₂, Ac-NHCH(CH₂Ph)-B(OH)₂, MeOCO-FGA-NHCH(Ph)CH₂-B(OH)₂, Z-GA-N(CH₃)CH(Ph)CH₂-B(OH)₂- wherein Z is benzyloxycarbonyl, Me is methyl and Ac is acetyl.

Alternatively, the serine protease inhibitor may be a peptide aldehyde with the formula:
P-O-(Aⁱ-X')ₙ-Aⁿ⁺¹-Q
wherein Q is hydrogen, CH₃, CX₃, CHX₂, or CH₂X, wherein X is a halogen atom;
wherein one X' is the "double N-capping group" CO, CO-CO, CS, CS-CS or CS-CO,
most preferred ureido/"urea" (CO), and the other X' es are nothing,
wherein n = 1-10, preferably 2-5, most preferably 2,
wherein each of Aⁱ and Aⁿ⁺¹ is an amino acid residue having the structure: -NH-CR-CO- for a residue to the right of X= -CO-, or -CO-CR-NH- for a residue to the left of X= -CO-wherein R is H- or an optionally substituted alkyl or alkylaryl group which may optionally include a hetero atom and may optionally be linked to the N atom. Preferred amino acids at the C-terminal (Aⁿ⁺¹) are L or D-amino acids with an optionally substituted aliphatic or aromatic side chain, preferably D- or L-Tyr (p-tyrosine), m-tyrosine, 3,4-dihydroxyphenylalanine, Leu, Phe, Val, Gly, Met, Trp, Ile, Nva or Nle. Preferred amino acid residues Ai (i<=n) to the right of X=-CO are those with small optionally substituted aliphatic side chain, preferably selected from Ala, Cys, Gly, Leu, Arg, Pro, Ser, Ile, Thr, Val, Nva, or Nle,
wherein P is hydrogen or any C-terminal protection group.

Examples of such peptide aldehydes include α-MAPI, β-MAPI, F-urea-RVY-H, F-urea-GGY-H, F-urea-GGF-H, F-urea-GAF-H, F-urea-GAY-H, F-urea-GAL-H, F-urea-GA-Nva-H, F-urea-GA-Nle-H, Y-urea-RVY-H, Y-urea-GAY-H, F-CS-RVF-H, F-CS-RVY-H , F-CS-GAY-H , Antipain, GE20372A, GE20372B, Chymostatin A, Chymostatin B, and Chymostatin C. Further examples of peptide aldehydes are disclosed in WO 2009/118375 and PCT/EP2009/053580, WO 94/04651, WO 98/13459, WO 98/13461, WO 98/13462, WO 2007/145963, (P&G) hereby incorporated by reference.

As another alternative, the serine protease inhibitor may be a borate such as borax. It may also be a boronic acid derivative having the formula B(OH)₂-C₆H₄-R wherein -C₆H₄-has bonds attached in the m- or p-position, and R is selected from the group consisting of CHO, , COOH, C₁-C₆ alkyl substituted CO-C₁-C₆ alkyl, C₁-C₆ alkenyl and substituted C₁-C₆ alkenyl, X (halogen),; most preferred is 4-formyl phenylboronic acid (4-FPBA) and borax.

As another alternative the serine protease inhibitor may be an aliphatic aldehyde, phosphonate or boronic acid with the structure:
D²-D¹-W
   - W is an aldehyde (CHO) or aldehyde analogue (COCX₃, COCHX₂, COCH₂X or COCH₃, where X is a halogen atom), a phosphorous derivative or a boronic acid (B(OH)2).
   - D¹ may be a hydrophobic chain of at 5-15 atoms, preferably carbon, oxygen and nitrogen, most preferred 6-8 atoms, if branched at position 3-8 (counting from W), then it must be with small substituens, preferably hydrophobic. If branched at position 1 or 2 (counting from W) then it must be an optionally substituted phenyl or aliphatic chain.
   - D² if present, may be H, a protection group, any amino acid, a small or a bulky group.

Examples of such inhibitors are Z-NH-(CH₂)₇-CHO, Z-NH-(CH₂)₆-CH(CH₂Ph)-CHO and Z-NH-(CH₂)₆-CH(CH₂C₆H₄-pOH)-CHO.

Further examples of inhibitors are described in WO 95/25791, W09813459, W09813461, W09813462, W02007145963 and W02007141736.

### Serine hydrolase composition

The composition comprises a serine hydrolase acting on ester bonds and a serine protease inhibitor, and it does not contain a protease. It may be in granular or liquid form. It may be a detergent , or a non-detergent, i.e. a serine hydrolase formulation without a surfactant.

### Non-detergent composition

For a non-detergent composition, the serine hydrolase acting on ester bonds may be a lipase present in an amount of 5-40 mg of enzyme protein per g, more preferred 10-30 mg enzyme protein/g. If the serine protease inhibitor is a peptide aldehyde or analogue it may be present in an amount of 0.01%-1%, more preferred 0.02%-0.5% or at a molar ratio of inhibitor:lipase of 0.5:1 to 20:1, more preferred 1:10 and even more preferred 1:5. If the serine protease inhibitor is a boric acid derivative (ex borax or substituted/unsubstituted phenylboronic acid derivatives) the inhibitor is present in an amount of 0.5-20%; more preferred 1%-10%.

Other ingredients in a non-detergent liquid compositions may be - but not limited to - polyols or mixtures of polyols (ex sorbitol, glycerol, monopropylene glycol, etc.)in 1-60%, small amounts of non-ionic surfactants (ex softanol) 0-5%, calcium ions 0.001%-0.5%, water, and preservatives (ex proxel, 2-phenoxyethanol, etc).

The serine protease inhibitor may be used to stabilized a lipase in a particle for triggered release of a rinse benefit agent comprising a lipase substrate, e.g. as described in W02009/118329.

### Detergent composition

For a liquid or granular detergent, the non-detergent composition described above may be added in an amount of 0.01%-5%, more preferred 0.1-2.5%, or the enzyme a and athe protease inhibitor may be added separately. The serine hydrolase acting on ester bonds may be a lipase present in an amount of 0.1-2 mg enzyme protein per g detergent. If the serine protease inhibitor is a peptide aldehyde or analogue it may be present in an amount of 0.01 ppm-0.02%, more preferred 0.5 ppm-0.01 %, even more preferred 0.8 ppm-40 ppm, or at a molar ratio of inhibitor:lipase of 0.5:1 to 20:1, more preferred up to 10:1 and even more preferred up to 5:1. If the serine protease inhibitor is a boric acid derivative (ex borax or substituted/unsubstituted phenylboronic acid derivatives) the inhibitor is present in an amount of 0.1 ppm-3%, more preferred 1 ppm-1 %, even more preferred 5 ppm-0.5%, most preferred 30 ppm-0.2%. Other ingredients may include chelating agents, solvents, colorants, and perfumes. If the detergent is a liquid, then pH is between 5-10, more preferred between 7-10 or 7-9.

The liquid detergent may be aqueous, typically containing 5-95% water, e.g. 5-15% or 20-70% water and 0-20% organic solvent (hereinafter, percentages by weight).

The detergent comprises a surfactant which may be anionic, non-ionic, cationic, amphoteric or a mixture of these types. The detergent will usually contain 5-30% anionic surfactant such as linear alkyl benzene sulphonate (LAS), alpha-olefin sulphonate (AOS), alcohol ethoxy sulphate (AES) or soap. It may also contain 3-20% anionic surfactant such as nonyl phenol ethoxylate or alcohol ethoxylate.

The pH (measured in aqueous detergent solution) will usually be neutral or alkaline, e.g.. The detergent may contain 1-40% of a detergent builder such as zeolite, phosphate, phosphonate, citrate, NTA, MGDA, GLDA, EDTA or DTPA, or it may be unbuilt (i.e. essentially free of a detergent builder). It may also contain other conventional detergent ingredients, e.g. fabric conditioners, foam boosters, bactericides, optical brighteners and perfumes.

The detergent composition may be a fabric cleaning compositions, hard surface cleansing compositions, light duty cleaning compositions including dish cleansing compositions and automatic dishwasher detergent compositions.

The liquid detergent composition may comprise from about 0.0001 % to about 10%, more particularly from about 0.00015% to about 1 %, and most particularly from about 0.001 % to about 0.1% of the inhibitor

Thus, a stabilized liquid enzyme formulation typically contains 0.5-20% by weight, particularily 1-10% by weight, of enzyme protein (total of protease and optional second enzyme) and 0.01%-10% of the inhibitor, more particularly 0.05-5% by weight and most particularly 0.1 %-2% by weight of the inhibitor.

A liquid detergent formulation will typically contain 0.04-400 micromolar enzyme (or 1-10000 mg EP/L), more particularily 0.16-160 micromolar enzyme (4-4400 mg EP/L) and most particularly 0.8-80 (20-2200 mg EP/L) and about 1-20 times more of the inhibitor, most particularly about 1-10 times more of the inhibitor.

The liquid detergent composition may contain water and other solvents as carriers. Low molecular weight primary or secondary alcohols exemplified by methanol, ethanol, propanol, and iso-propanol are suitable. Monohydric alcohols are preferred for solubilizing surfactants, but polyols such as those containing from about 2 to about 6 carbon atoms and from about 2 to about 6 hydroxy groups (*e.g.*, 1,3-propanediol, ethylene glycol, glycerol, and 1,2-propanediol) can also be used. The compositions may contain from about 5-90%, 10-50% 2-50%; 2-25% of such carriers.

The detergent compositions herein will preferably be formulated such that during use in aqueous cleaning operations, the wash water will have a pH between about 6.8 and about 11. Finished products are typically formulated at this range. Techniques for controlling pH at recommended usage levels include the use of, for example, buffers, alkalis, and acids. Such techniques are well known to those skilled in the art.

When formulating the hard surface cleaning compositions and fabric cleaning compositions of the present invention, the formulator may wish to employ various builders at levels from about 5% to about 50% by weight. Typical builders include the 1-10 micron zeolites, polycarboxylates such as citrate and oxydisuccinates, layered silicates, phosphates, and the like. Other conventional builders are listed in standard formularies.The invention is particularly applicable to the formulation of liquid detergents where enzyme stability problems are pronounced.

The detergent may in particular be formulated for laundry, dishwashing or hard surface cleaning, e.g. for floor cleaning, carwash, industrial and institutional laundry, or washing of wool and silk textiles. The detergent may be formulated as described in US2006247150, W02005040320, US2005020466, US2009217463, W02008010920, W02007087259, W09600277, W09511292, EP430315, DE10023580, W09617052, W02009107091, W02008010920, W02007087259, W09600277, JP2006131659, JP2004204084 JP2004203918, W02009060966, W02006050308 and JP63260997.

### Use of serine hydrolase acting on ester bonds

The serine protease inhibitor may be used to stabilize the serine hydrolase acting on ester bonds in the absence of a protease. Thus, the inhibitor may be used in the manufacture of the serine hydrolase, e.g. by adding it during fermentation, recovery or during formulation of the serine hydrolase acting on ester bonds, e.g. as described in WO95/25791. The inhibitor may also be used in a lipid-based drug delivery system containing phospholipase A2, e.g. as described in W0200158910.

### EXAMPLES

### Example 1: Liquid lipase formulation

Lipoclean formulations, per se (LCLU/g) residual activities:
The Lipase was formulated from crude concentrate with standard formulation ingredients (sorbitol and glycerol) with and without the serine protease inhibitor (2% enzyme protein, 0.08% Z-GAY-H added with a 1% DMSO solution). The solution was split into separate vials which were incubated at different temperature and times and compared in activity with a frozen reference sample frozen directly after mixing. The lipase activity was measured by ester bond hydrolysis in the substrate PNP-Palmitale (C:16) and releases PNP which is yellow and can be detected at 405 nm.

| | 2 weeks, 50°C | 4 weeks, 40°C | 4 weeks, 50°C |
|---|---|---|---|
| No inhibitor | 54% | 45% | 40% |
| 0,08% serine protease inhibitor | 71% | 70% | 56% |

## Claims

1. A composition which comprises a serine hydrolase acting on ester bonds and a serine protease inhibitor, and which does not comprise a protease.

2. The composition of claim 1 which is in the form of granules or a liquid.

3. The composition of claim 1 or 2 which is a detergent composition further comprising a surfactant.

4. The composition of claim 1 or 2 which does not comprise a surfactant.

5. The composition of any preceding claim wherein the serine hydrolase is a lipase.

6. The composition of the preceding claim wherein the lipase has an amino acid sequence which is at least 80% identical to *Thermomyces lanuginosus* lipase, *Candida antarctica* lipase A or *Candida antarctica* lipase B.

7. The composition of any of claims 1-4 wherein the serine hydrolase is a phospholipase A2.

8. The composition of any preceding claim wherein the serine protease inhibitor has the general formula: A⁴-A³-A²-A¹, where
o A¹ is an aldehyde (CHO) or aldehyde analogue (COCX₃, COCHX₂, COCH₂X or COCH₃, where X is a halogen atom), a phosphorous derivative or a boronic acid (B(OH)₂)
o A² may be an aromatic, or an aliphatic part. If A² is an amino acid, then the C-terminal carbon is included in A¹ described above.
o A³ if present may a non-bulky linker, preferably hydrophobic.
o A⁴ if present may be a protection group (e.g. an N-capping group as described below), any amino acid, a small or a bulky group.

9. The composition of any preceding claim wherein the serine protease inhibitor is a peptide aldehyde.

10. The composition of any preceding claim wherein the peptide aldehyde has the formula B²-B¹-B⁰-R wherein:
R is hydrogen, CH₃, CX₃, CHX₂, or CH₂X, wherein X is a halogen atom;
B⁰ is a single amino acid residue;
B¹ is a single amino acid residue; and
B² consists of one or more amino acid residues (preferably one or two), optionally comprising an N-terminal protection group.

11. The composition of any preceding claims 1-7 where the serine protease inhibitor has the formula: P-O-(Aⁱ-X')ₙ-Aⁿ⁺¹-Q
o wherein Q is hydrogen, CH₃, CX₃, CHX₂, or CH₂X, wherein X is a halogen atom;
o wherein one X' is the "double N-capping group" CO, CO-CO, CS, CS-CS or CS-CO, most preferred ureido/"urea" (CO), and the other X' es are nothing,
o wherein n = 1-10, preferably 2-5, most preferably 2,
o wherein each of Aⁱ and Aⁿ⁺¹ is an amino acid residue having the structure:
o -NH-CR-CO- for a residue to the right of X= -CO-, or
o -CO-CR-NH- for a residue to the left of X= -CO-
o wherein R is H- or an optionally substituted alkyl or alkylaryl group which may optionally include a hetero atom and may optionally be linked to the N atom.

12. The composition of any of claims 1-7 wherein the serine protease inhibitor is a borate or a boronic acid derivative having the formula B(OH)₂-C₆H₄-CO-R wherein -C₆H₄- has bonds attached in the p-position, and R is selected from the group consisting of hydrogen, hydroxy, C₁-C₆ alkyl substituted C₁-C₆ alkyl, C₁-C₆ alkenyl and substituted C₁-C₆ alkenyl, e.g. 4-formylphenyl-boronic acid (4-FPBA).

13. A compound having the structure Z-GGF-H, Z-GGG-H, Z-GAG-H, Z-NH-(CH₂)₇-CHO, Z-NH-(CH₂)₆-CH(CH₂Ph)-CHO or Z-NH-(CH₂)₆-CH(CH₂C₆H₄-pOH)-CHO.

14. Use of a serine protease inhibitor to stabilize a serine hydrolase acting on ester bonds in the absence of a protease.
